# EUROPEAN PATENT APPLICATION

(11) **EP 2 111 820 A1**
(43) Date of publication of application: **28.10.2009**
(21) Application number: 07704731.4
(22) Date of filing: 17.01.2007
(51) Int. Cl.: A61F 2/16

(54) **CONTAINER FOR AN INTRAOCULAR LENS AND METHOD FOR APPLYING AN INTRAOCULAR LENS**

(71) Applicant: Ajl, S.a., 01510 Miñano Alava (ES)
(72) Inventor: SALAZAR SALEGUI, Pedro Jose, 01510 Miñano (Alava) (ES); LARRA MATEOS, Maria Eva, 01510 Miñano (Alava) (ES); IPIÑAZAR ALONZO, Enrique, E-20009 San Sebastian (ES); ELIZETXEA, Ezeiza, Cristina, E-20009 San Sebastian (ES); ARRIZABALAGA CANELLADA, Igor, E-20009 San Sebastian (ES)
(74) Representative: Carpintero Lopez, Francisco
(86) International application number: PCT/ES2007/000017
(87) International publication number: WO 2008/087226

(57) **Abstract**

The invention relates to a container for an intraocular lens, comprising a container body (1) and a lid (2) associated with a securing device (3) configured to secure an intraocular lens (4). Two parts (3A, 3B) of the securing device are mobile with respect to one another, allowing the lens to fold. The folded lens is located in a housing space (31) that is in communication with two openings (32, 33), one (33) of which is configured to receive a driving element (51) for moving the lens through the second opening (32) to an exit (35).

The invention also relates to a container and injector assembly, and to a method for applying an intraocular lens.

## Description

### Technical Field of the Invention

The invention is comprised in the field of containers or vials for intraocular lenses.

### Background of the Invention

An intraocular lens (IOL) comprises a central optical part (the lens *per se)* with a reduced size (usually with a diameter between 5 and 7 mm) and fixing means (haptics), through which the lens is fixed within the capsular bag. These lenses are implanted, for example, in people with cataracts, and the implantation is performed through a small incision in the ocular wall. In order for the incision that is to be made to have the smallest possible size, intraocular lenses are currently manufactured with materials that allow them to be folded, such that the lens is inserted in the eye folded, and recovers its original shape once implanted.

Until they are implanted, intraocular lenses are usually stored in a container (vial) filled with a saline solution, or in sterile holders in which the intraocular lens is in a dry state, i.e., without saline solution (examples of this type of holder are described in, for example, US-A-5199559 and US-A-4736836). These holders (i.e., those which do not contain a saline solution) have an inner housing with a configuration which adapt to the configuration of the lens in a very exact manner so that the lens is perfectly supported and does not deteriorate due to the movement inside the holder, since it is not floating in a liquid. This means that each manufacturer uses a different holder, according to the lenses he uses.

Vials with saline solution have the advantage that the lens is in a wet state, similar to the state it will be in within the eye, such that the lens has suitable properties for its immediate implantation.

To implant the lens, the surgeon must perform the following steps:
- Take the lens out of the vial with the saline solution.
- Fold the lens. For this operation, special cartridges are normally used in which the lens is deposited for the folding thereof.
- Inserting the lens in the eye through the small hole made. For this operation, special syringes are used which have a plunger pushing the lens into the capsular bag. These syringes are usually coupled to the cartridges in which the lens is previously folded.

All these operations involve complicated and precise handling operations, since the surgeon must perform them without touching the lens with his hands and without the lens coming into contact with elements that can contaminate it. It is therefore advisable to simplify the process.

The operation of taking the lens out of the vial must be done with special tweezers and is complicated since the lens is virtually transparent and its position in the vial is difficult to locate. Furthermore, the lens is often floating in the vial, so it is difficult to grasp it with the tweezers. The saline solution content can also be emptied out, but the lens thus usually remains adhered to the wall of the vial, therefore it is not easy to grasp it in this case either.

Several vials are known which have a device which facilitates taking the lens out, such as, for example, those described in US-A-4423809, US-B-6360883 or WO-A-88/01142.

There are also vials having devices which allow folding the lens, such that the surgeon does not have to perform this operation. US-B-6183513, EP-A-1421917 and BE-A-1011907 describe systems of this type. These documents do not describe how the lens is inserted or implanted, although it is probable that the intention is for the surgeon to take the folded lens and insert it in the eye with forceps or tweezers or the like. This operation requires a great deal of precision.

It is most common to use special cartridges to fold the lens, such as for example the one described in WO-A-96/11649. This cartridge has an injection tube which is partially inserted in the eye and through which the folded intraocular folded lens moves (to that end a syringe or injector device is used), and a chamber for positioning the intraocular lens. The intraocular lens is deposited in this chamber (with tweezers or the like) unfolded (the lens is taken out of the corresponding vial), and is formed by two semi-cylindrical parts connected to one another through a longitudinal line functioning as a hinge, such that when the two semi-cylindrical parts are lowered, a cylindrical housing is formed inside which the intraocular lens is folded, i.e., the lowering of the two semi-cylindrical parts causes the folding of the intraocular lens. This cylindrical housing is aligned and in communication with the injection tube. To facilitate this lowering, the mentioned semi-cylindrical parts have wing-like extensions which abut with one another for folding the lens.

Once the lens is folded by means of the cartridge, a syringe or injector is normally used which has a cylindrical body in which the folded cartridge (with the folded lens) is inserted and a plunger which, when it moves within the cylindrical body, moves the lens from its housing of the cartridge, through the injection tube, into the eye.

A syringe or injector of this type is observed, for example, in US-A-2003/0050647. Other documents relating to cartridges, syringes or similar injection devices are described and illustrated in, for example, EP-A-1440674, US-A-5716364, WO-A-96/20662, WO-A-99/62436, WO-A-95/29648, WO-A-2004/091447, US-B-6723104, JP-A-2004-344213, US-A-5728102, US-A-6143001, US-B-6398789, US-A-6083261, US-B-6712848, US-A-5928245, WO-A-03/015657, US-A-2004/0199173 or WO-A-2004/105649.

WO-A-2004/108018 describes a storage case for an intraocular lens which allows automatically loading the lens in the cartridge, without having to manually handle the lens. This case is a special case (in a wet situation) which only serves for one type of intraocular lens.

It has therefore been considered that the use of three different devices (such as the vial, the cartridge and the injector) represents a problem, since they require a more or less complex handling operation, always include a step for transferring the lens, which must be manually performed by the surgeon, using tweezers or the like. No device is known which allows performing all the operations without the need to manually handle the lens.

### Description of the Invention

A first aspect of the invention relates to a container for an intraocular lens, comprising a container body (for example, of glass or of plastic) and a lid (for example, of any type of plastic or the like, for example, obtained by injection molding) configured to close an opening in the container body. The lid is associated with a securing device configured to secure an intraocular lens.

The securing device comprises at least a first part and a second part which are in contact with the lens when the securing device secures the lens.

According to the invention, said first part and second part are at least partially mobile with respect to one another such that the first part can pass (for example, pivoting with respect to the second part) from a first position relative to the second part, in which the lens is not folded, to a second position relative to said second part, in which the lens is folded and housed in a housing space of the securing device (for example, a housing space laterally demarcated by said first part and by said second part).

Furthermore, according to the invention, the securing device is configured such that said housing space is in communication with two openings, such that the housing space is located between said openings, a first of said openings being configured to receive a driving element (for example, the plunger of a syringe) for moving the lens through the second opening, and the second opening being associated with an injection channel configured so that the lens passes through said channel, from the housing space to an exit.

The securing device itself, which can secure the lens in an "unfolded" manner in the container or vial, thus serves to fold the lens before its application, and to keep it folded until an injector pushes it out. Any need for a manual handling operation of the lens during the process of removing it from the container and the final application thereof is thus avoided.

The lid can comprise a main part in which the securing device is coupled, and a shutter configured such that it can turn with respect to the main part. This shutter can serve to pass or drive the first part of the securing device from its first position and to its second position with respect to the second part of the securing device. The shutter can be coupled (for example, by screwing) in a hole in the main part of the lid, for example, in a central hole, closing said hole, with the possibility of leaving it clear so that an injector device, for example, a syringe, is coupled therein.

The shutter can have one or more flaps to facilitate the turning of the shutter by a user.

The main part of the lid can comprise at least one coupling element configured to secure the securing device (for example, the second part of the securing device), such that the securing device can be coupled to the lid. The coupling element can comprise a guide channel (or several guide channels) to receive the securing device. The connection between the coupling element (or the coupling elements) and the securing device can be carried out in any suitable manner, for example, by grooving and tonguing or the like.

On the other hand, the main part of the lid can comprise a threaded part configured to be screwed in a complementary threaded part of the container.

The first part of the securing device can be coupled to the shutter, and the second part of the securing device can be coupled to the main part of the lid, such that said first part can pass from said first position to said second position with respect to the second part, by means of one turn of the shutter with respect to the main part of the lid. The lens can thus pass from its unfolded configuration to its folded configuration by means of a simple turn of the shutter with respect to the main part of the lid. This turn can correspond to a first phase of an unscrewing of the shutter with respect to the main part of the lid.

The first part of the securing device can be provided with a coupling element for coupling to the shutter, for example, with a protruding part configured to enter a hole in the shutter. This protruding part can serve to force the first part of the securing device to follow the turning movement of the shutter, pivoting with respect to the second part of the securing device, until reaching the second position discussed above. The protruding part can be configured to be broken and/or separated from the first part of the securing device if the user continues turning the shutter (with certain force) once the first part of the securing device has reached the second position, releasing the shutter from its coupling to said first part, such that the shutter can be completely unscrewed to then be removed from the main part of the lid, leaving the corresponding hole in the main part of the lid clear.

At least one of said first part and second part can be provided with at least one retaining element (for example, a flange or the like) configured to cooperate with a complementary retaining element (for example, a window or the like) in the other one of said first part and second part, to lock said first part in said second position with respect to the second part. Thus, once the first part reaches the second position, it is retained therein.

The first part and the second part of the securing device can be connected to one another by a weakened area (for example, an area of less thickness, or a partially die-cut area) configured to function as a hinge between said first part and second part.

The openings between which the housing space is located can be axially opposite openings.

The securing device can be formed by a single piece element obtained by injection molding. The securing device can be obtained together with the main part of the lid, or as an independent element which is subsequently coupled to the main part of the lid and to the shutter.

The shutter can be configured to be able to be removed from the main part of the lid once said first part of the securing device is in said second position relative to the second part of the securing device, to provide access to an injector which can be coupled in the hole left clear by the shutter.

The lid can be configured such that the shutter can be removed to allow an injector being coupled in the lid, such that a driving element (for example, a plunger or the like) of the injector can drive an intraocular lens housed in the housing space, towards the exit of the securing device.

The container can furthermore contain an intraocular lens (secured by the securing device) and a saline solution.

Another aspect of the invention relates to an assembly or kit comprising a container according to what has been described above, and an injector configured to be coupled in the lid (for example, in a hole previously occupied by the shutter, for example, by means of screwing), such that a driving element of the injector can drive an intraocular lens housed in the securing device. In other words, the injector could be configured to be coupled in a hole in the main part of the lid once the shutter is removed. In such case, said hole in the main part could be aligned with the two openings and with the housing space of the securing device.

Another aspect of the invention relates to a method for applying (for example, in a human being's eye) an intraocular lens housed in a container with a lid connected to a securing device securing the lens inside the container (for example, in a container such as the one that has been described above). The method comprises the steps of:
a) turning a shutter associated with the lid, driving a first part of the securing device from a first position to a second position with respect to a second part of the securing device, locking said first part in said second position (for example, by means of one or more locking flanges), such that the lens is folded in a housing space of the securing device;
b) removing the shutter such that a hole in the lid is clear;
c) coupling an injector in said hole; and
d) activating the injector such that a driving element of the injector (for example, a plunger) enters the securing device, ejecting the lens through an exit of the securing device.

In step (b), the shutter can be removed, for example, by means of unscrewing, causing a protrusion or the like of the securing device housed inside a hole or the like of the shutter to break.

The first part of the securing device can be retained in said second position by means of at least one retaining element forming part of the securing device.

The method can additionally comprise the step of removing the lid from the container before step a), between step a) and step b), between step b) and step c), or between step c) and step d).

### Description of the Drawings

To complement the description and for the purpose of aiding to better understand the features of the invention according to a preferred practical embodiment thereof, a set of drawings is attached as an integral part of the description in which the following has been depicted with an illustrative and nonlimiting character:
Figure 1 shows a perspective view of a securing device according to a possible embodiment of the invention.
Figures 2-14 show views, from different angles, of the lid and of associated elements during several phases of a process for applying an intraocular lens according to a possible embodiment of the invention.
Figures 14-15 show two perspective views of a container according to a possible embodiment of the invention.

### Preferred Embodiment of the Invention

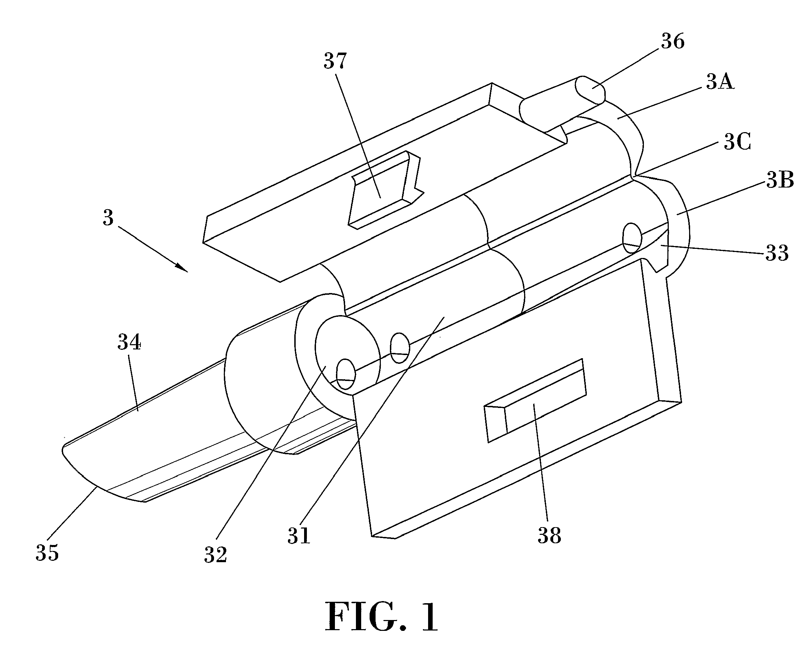

Figure 1 shows a securing device 3 according to a possible embodiment of the invention. In this case, the securing device comprises a body of a plastic material, obtained as a single piece in an injection molding process. The device comprises a first part 3A connected to a second part 3B through a weakened area 3C (with a smaller thickness than the general thickness of the first part 3A and of the second part 3B), such that this weakened area allows the first part 3A to pivot with respect to the second part (and vice versa). A lens can be coupled to said first part 3A and second part 3B, and the lens can be folded by pivoting the first part 3A towards the second part 3B, until it is bent, housed in a housing space 31 inside the securing device. When the first part 3A abuts against the second part 3B, a retaining element (in this case, a flange 37) of the first part 3A is coupled in a complementary retaining element (in this case, the window 38) of the second part 3B, such that the first part is retained in this position, i.e., in the position corresponding to the folded lens within the retaining device 3.

The housing space 31 is in communication at one end with an opening 33 through which a driving element (for example, the plunger of a syringe) can enter, and at the axially opposite end, with a second opening 32 that is in communication with an injection channel (which runs through the injection tube 34 and which comes out in the exit 35 of said ejection tube). The lens housed in the housing space 31 can thus pass through the channel of the injection tube 34 and exit through the exit 35 by inserting a plunger through the first opening 33. The lens can thus be inserted in the eye by inserting the injection tube in an eye.

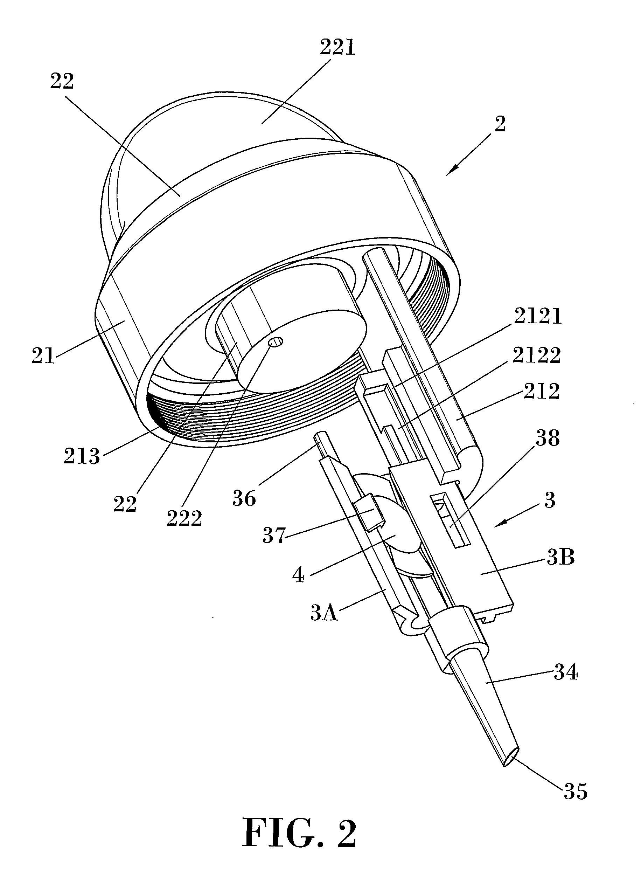

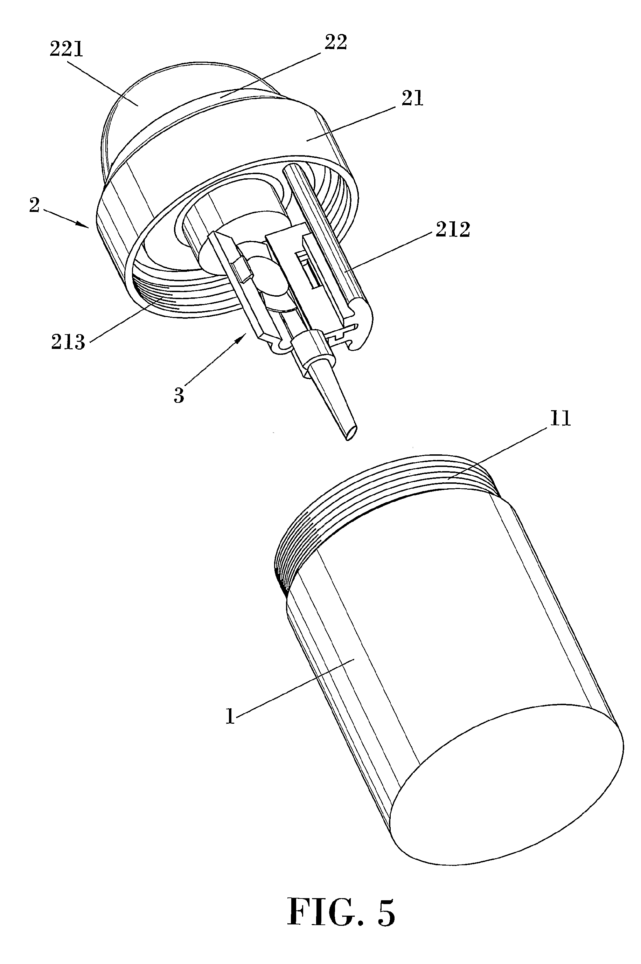

In Figure 2, which shows a perspective view of the lid 2, it can be seen how the securing device 3 of Figure 1 is coupled to a main part 21 of the lid 2. This main part 21, which can be of plastic and be formed by a single piece obtained by molding, comprises a coupling element (212) provided with guide channels (2121, 2122) configured so that the securing device 3 can be coupled to the main part 21 of the lid, by means of a sliding movement substantially perpendicular to the lid. The retention can be caused by means of any conventional system, for example, by grooving and tonguing. It can also be possible to obtain the lid (or at least the main part of the lid 21) and the securing device as a single piece body by means of, for example, injection molding. The main part of the lid also comprises a threaded part 213 which serves for screwing the lid to the container body 1 of (see Figure 5, for example, in which a complementary threaded area 11 of the container body can be seen).

On the other hand, in Figure 2 it can also be observed how the lid is provided with a shutter 22 which is screwed in a central hole of the main part 21 of the lid and which is provided with a flap 221 to facilitate the turning (screwed/unscrewing) of the shutter. Furthermore, it can be observed how the shutter 22 is provided with a hole 222 to receive a protruding part 36 (a type of pin) associated with the first part 3A of the securing device. In Figure 2, it can be observed how the first part 3A is in a first position (or open position) with respect to the second part 3B, such that the lens 4 is not folded.

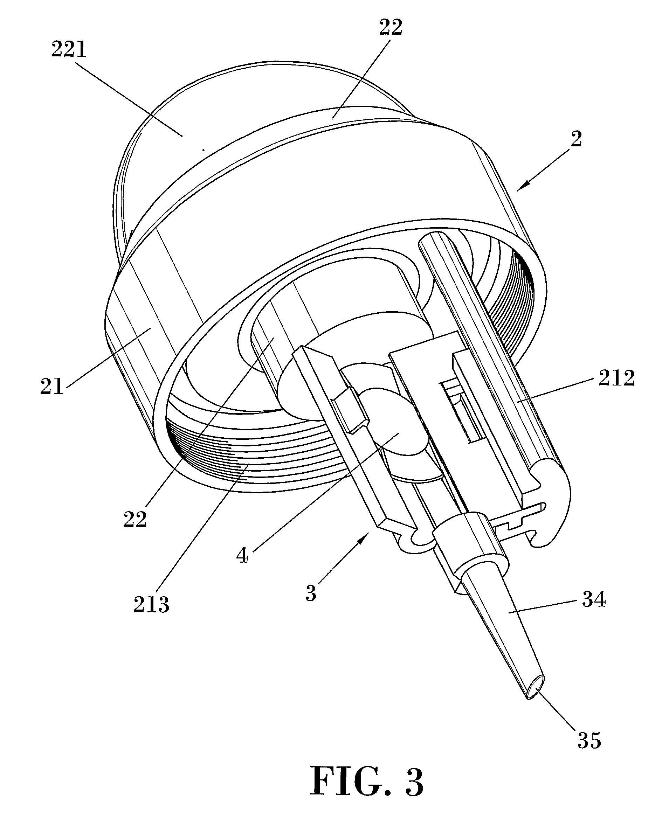

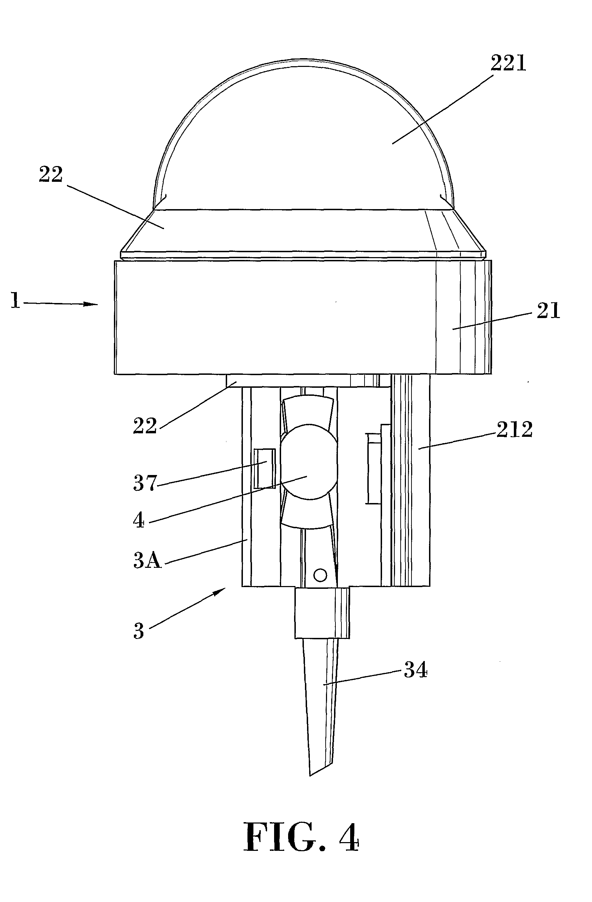

Figure 3 shows a view similar to that of Figure 2, but with the securing device 3 already completely coupled to the main part 21 of the lid 2, and with the protruding part or pin 36 inserted in the hole 222 of the shutter (see Figure 2). Figure 4 shows a side elevational view of the same arrangement.

Now the lid, with the lens 4 secured in the "unfolded" position, can be coupled to the body of the container, as illustrated in Figure 5 (for example, by means of screwing). The container body 1 can previously have been filled with a liquid, typically a saline solution, as is conventional. Once the lid has been applied and closed, a vial is provided which can be comfortably stored and distributed.

To "apply" an intraocular lens from the vial thus formed, the steps described below can be followed (the lid 2 can be removed from the container body at any time of the described process, for example, as a first step of the process or at any later time, prior to the final application of the lens).

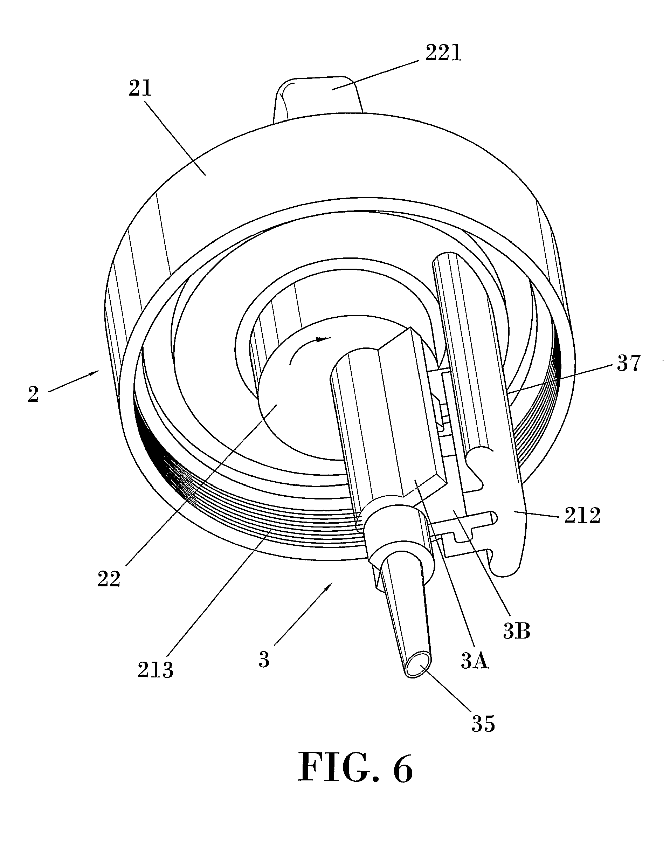

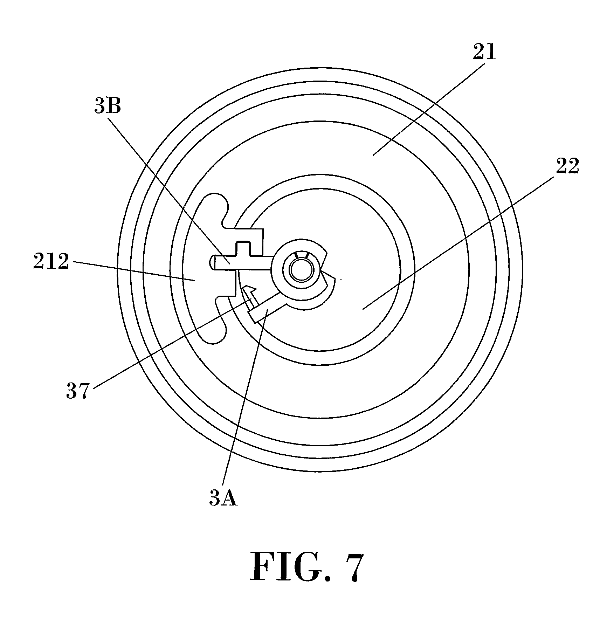

The process can begin by turning the shutter 22 in its "unscrewing" direction with respect to the main part 21 of the lid (see the arrow in Figure 6). The shutter thus "drives" the first part 3A of the securing device, "folding it" on the second part 3B, as is schematically illustrated in Figure 6. Figure 7 shows a corresponding bottom plan view of the lid. The first part 3A is driven due to the protruding part or pin 36 being housed in the hole 222 of the shutter, as has been explained above (see Figure 2). When pivoting the first part 3A from its first position (which can be seen in, for example, Figure 5) towards the second position, passing through the one that is seen in Figures 6 and 7, the lens is gradually folded inside the housing space 31.

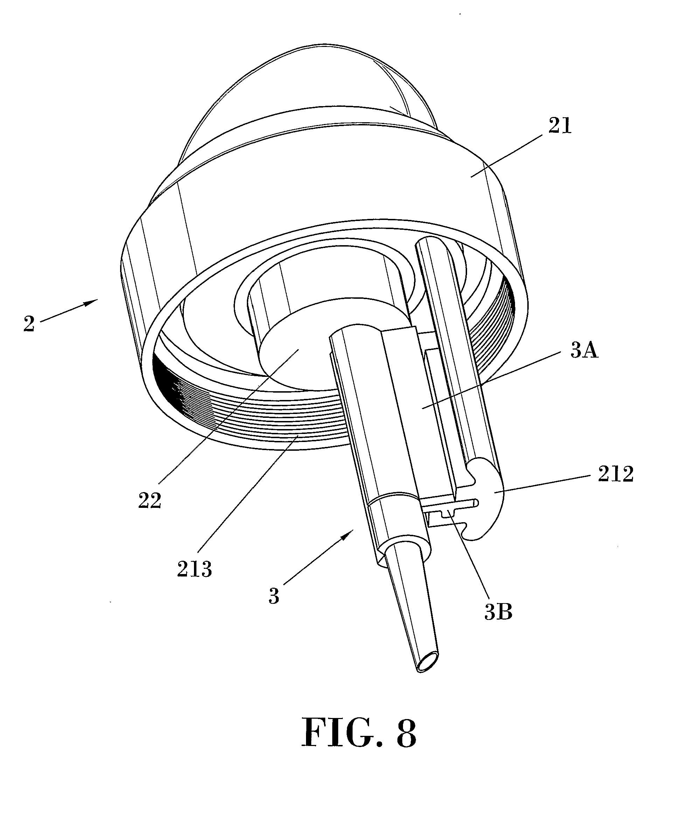

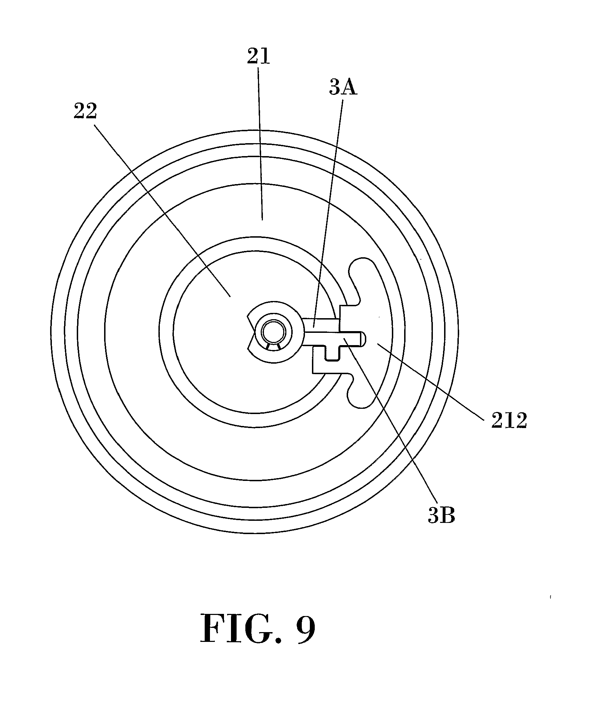

Once the first part 3A reaches its second position with respect to the second part 3B (as is illustrated in Figures 8 and 9), the first part 3A abuts against the second part 3B, and is retained since the flange 37 has penetrated the window 38 and has been fitted in a locking position. Now the lens (not illustrated in Figures 8 and 9) is housed in a folded manner inside the housing space 31 of the securing device 3.

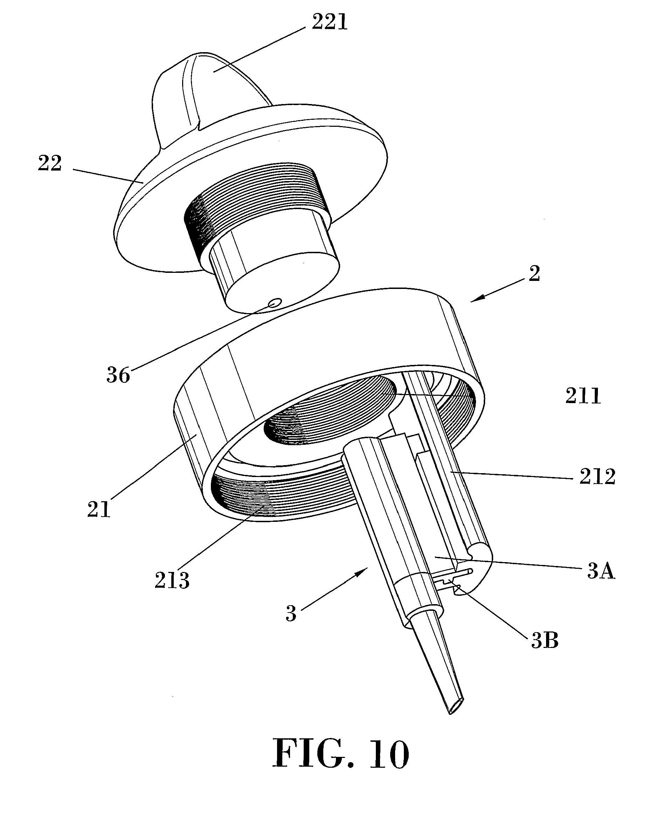

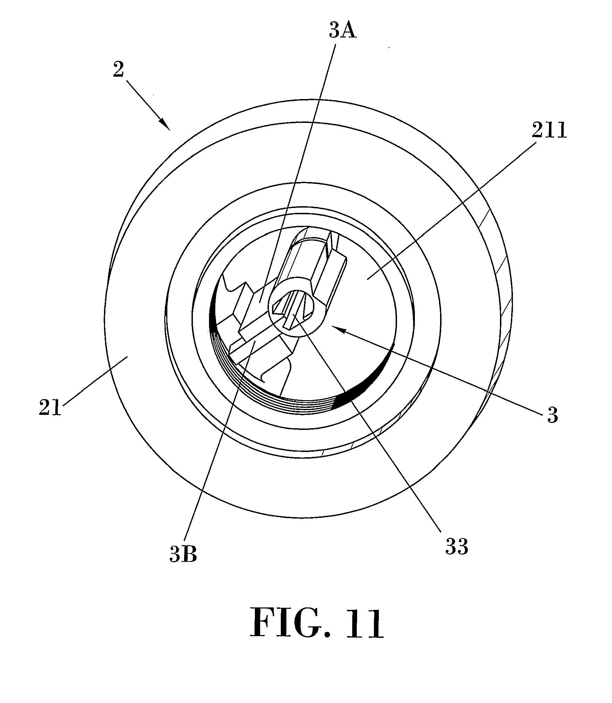

Next, the shutter 22 can be completely unscrewed, causing the pin 36 (which has been retained in the hole of the shutter in Figure 10) to break, and the shutter is removed, the threaded hole 211 being clear. The first part 3A of the securing device stays abutted against the second part 3B due to the retaining system discussed above. In Figure 11, the lid can be observed in an "upper" perspective view, and the hole or opening 33 formed between the parts 3A and 3B, through which a plunger or the like can be inserted to eject the lens through the axially opposite side of the securing device 3, can be seen.

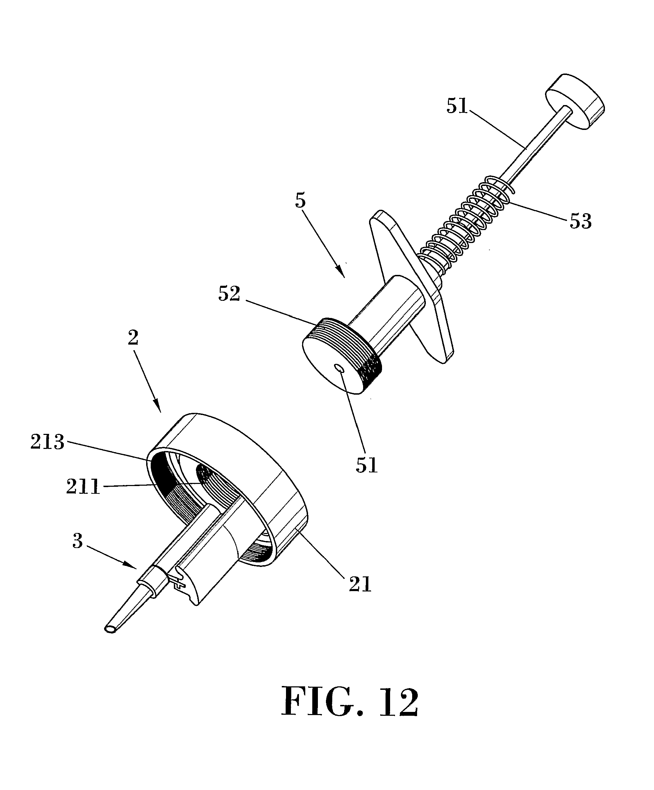

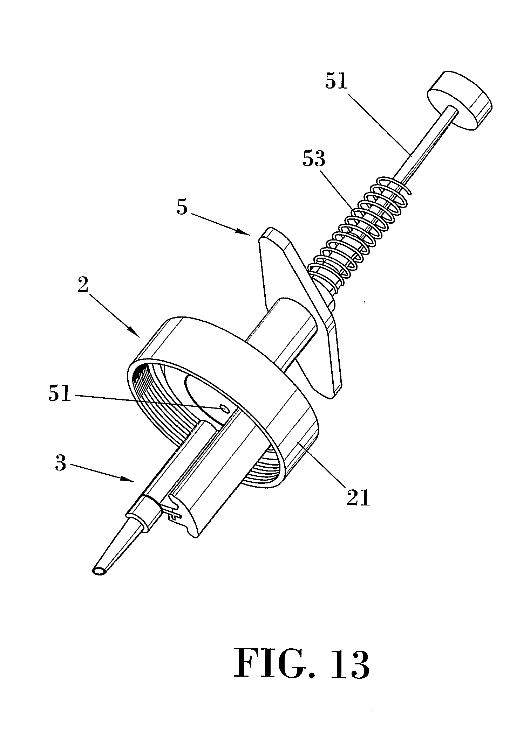

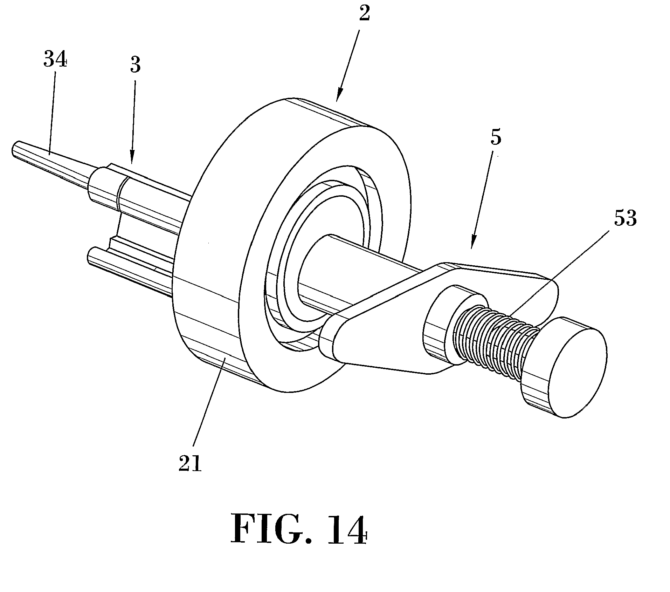

In a final step, it can have an injector, for example, in the form of a syringe 5 with its plunger 51, and with a threaded part 52 which is screwed in the same hole 211 in which the shutter was previously screwed, see Figures 12 and 13. To eject the lens (for example, to insert it in the eye of a patient), the injection tube 34 can be inserted in the eye and the plunger 51 driven (against the force of the spring 53), until reaching the position illustrated in Figure 14.

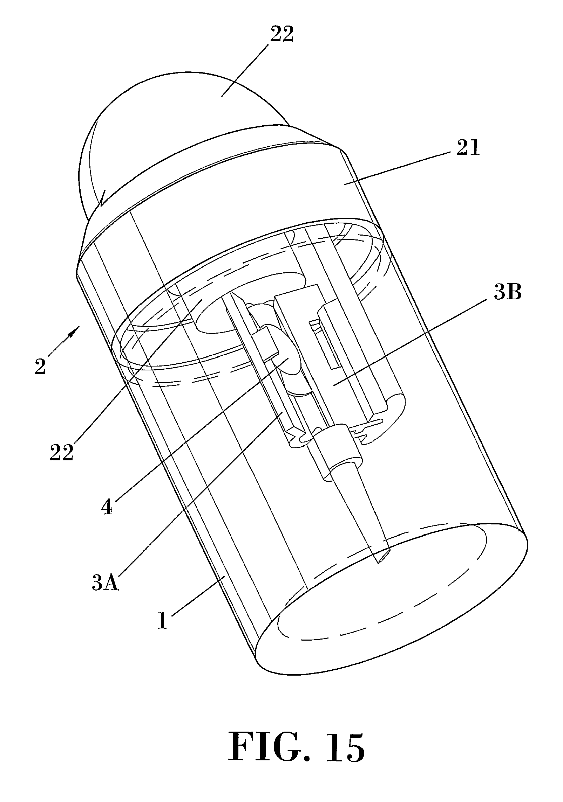

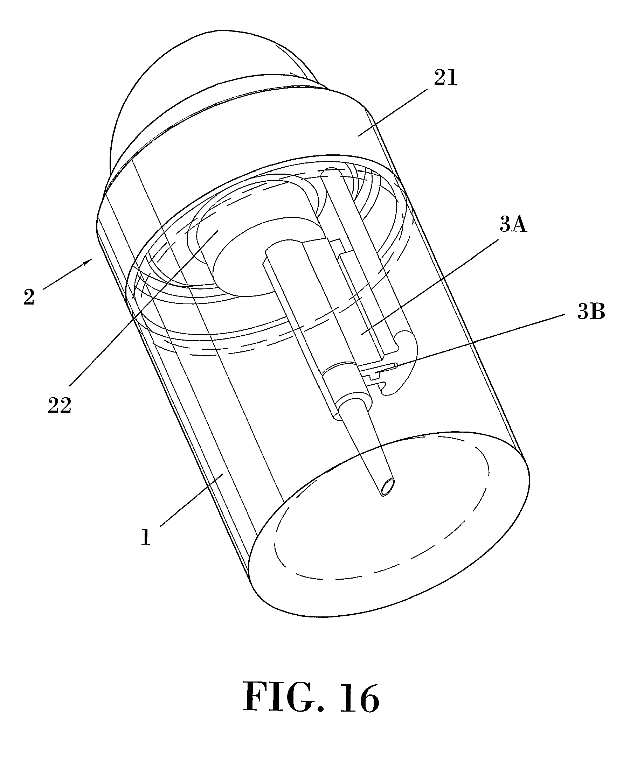

Figures 15 and 16 show two perspective views of the container. In Figure 15, the first part 3A is in its first position with respect to the second part 3B of the securing device, and in Figure 16 the first part has reached its second position.

In this text, the word "comprises" and variants thereof (such as "comprising", etc.) must not be interpreted in an excluding manner, i.e., they do not exclude the possibility that what is described may include other elements, steps etc.

On the other hand, the invention is not limited to the specific embodiments which have been described but also covers, for example, the variants which can be carried out by a person having average skill in the art (for example, with regard to the choice of materials, dimensions, components, configuration, etc.), within what is inferred from the claims.

## Claims

1. Container for an intraocular lens, comprising a container body (1) and a lid (2) configured to close an opening in the container body (1), the lid (2) being associated with a securing device (3) configured to secure an intraocular lens (4), said securing device comprising at least a first part (3A) and a second part (3B) which are in contact with the lens when the securing device secures the lens;
**characterized in that**
said first part (3A) and second part (3B) are at least partially mobile with respect to one another such that the first part (3A) can pass from a first position relative to the second part (3B), in which the lens is not folded, to a second position relative to said second part (3B), in which the lens is folded and housed in a housing space (31) of the securing device (3);
and **in that**
the securing device is configured such that said housing space (31) is in communication with two openings (32, 33), such that the housing space is located between said openings, a first (33) of said openings being configured to receive a driving element (51) for moving the lens through the second opening (32), and the second opening being associated with an injection channel configured so that the lens passes through said channel, from the housing space (31) to an exit (35).

2. Container according to claim 1, wherein the lid (2) comprises a main part (21) in which the securing device (3) is coupled, and a shutter (22) configured such that it can turn with respect to the main part (21).

3. Container according to claim 2, wherein said shutter (22) is coupled in a hole (211) in the main part (21) of the lid.

4. Container according to claim 3, wherein said shutter is screwed in said hole (211).

5. Container according to any of claims 2-4, wherein said shutter has at least one flap (221) to facilitate the turning of the shutter by a user.

6. Container according to any of claims 2-5, wherein the main part (21) of the lid comprises at least one coupling element (212) configured to secure the securing device (3).

7. Container according to claim 6, wherein said at least one coupling element (212) comprises at least one guide channel (2121, 2122) to receive the securing device (3).

8. Container according to any of claims 2-7, wherein the main part (21) of the lid comprises a threaded part (213) configured to be screwed in a threaded part (11) of the container.

9. Container according to any of claims 2-8, wherein the first part (3A) of the securing device is coupled to the shutter (22), and wherein the second part (3B) of the securing device is coupled to the main part (21) of the lid, such that said first part (3A) can pass from said first position to said second position with respect to the second part (3B) by means of one turn of the shutter (22) with respect to the main part (21) of the lid (2).

10. Container according to claim 9, wherein said first part (3A) is provided with a protruding part (36) configured to enter a hole (222) in the shutter (22).

11. Container according to any of claims 2-10, wherein at least one (3A) of said first part (3A) and second part (3B) is provided with at least one retaining element (37) configured to cooperate with a complementary retaining element (38) in the other one (3B) of said first part (3A) and second part (3B), to lock said first part (3A) in said second position with respect to the second part (3B).

12. Container according to claim 11, wherein said retaining element is a flange.

13. Container according to any of claims 2-12, wherein said first part (3A) and second part (3B) are connected to one another by a weakened area configured to function as a hinge between said first part (3A) and second part (3B).

14. Container according to any of claims 2-13, wherein the openings (32, 33) between which the housing space (31) is located are axially opposite openings.

15. Container according to any of claims 2-14, wherein the securing device (3) is formed by a single piece obtained by injection molding.

16. Container according to any of claims 2-15, wherein the shutter (22) is configured to be able to be removed from the main part (21) of the lid once said first part (3A) is in said second position relative to the second part (3B).

17. Container according to any of claims 2-16, wherein the lid (2) is configured such that the shutter (22) can be removed to allow an injector being coupled in the lid, such that a driving element of the injector can drive an intraocular lens, housed in the housing space (31), towards the exit (35).

18. Container according to any of the previous claims, further containing an intraocular lens (4) and a saline solution.

19. Assembly comprising a container according to any of the previous claims, and an injector (5) configured to be coupled in the lid (2), such that a driving element (51) of the injector can drive an intraocular lens (4) housed in the securing device.

20. Assembly according to claim 19, wherein the container is a container according to any of claims 2-17 and wherein the injector is configured to be coupled in a hole (211) in the main part (21) of the lid, once the shutter (22) is removed.

21. Method for applying an intraocular lens housed in a container with a lid connected to a securing device securing the lens inside the container, **characterized in that** it comprises the steps of:
a) turning a shutter (22) associated with the lid (2), driving a first part (3A) of the securing device from a first position to a second position with respect to a second part (3B) of the securing device, locking said first part in said second position, such that the lens (4) is folded in a housing space (31) of the securing device;
b) removing the shutter (22) such that a hole (211) of the lid is clear;
c) coupling an injector (5) in said hole (211);
d) activating the injector such that a driving element (51) of the injector enters the securing device (3), ejecting the lens through an exit (35) of the securing device.

22. Method according to claim 21, wherein in step (b), the shutter is removed by means of unscrewing it, causing a protrusion (36) of the securing device housed inside a hole (222) of the shutter (22) to break.

23. Method according to claim 21 or 22, wherein said first part is retained in said second position by means of at least one retaining element forming part of the securing device.

24. Method according to any of claims 21-23, additionally comprising the step of removing the lid from the container before step a).

25. Method according to any of claims 21-23, additionally comprising the step of removing the lid from the container between step a) and step b).

26. Method according to any of claims 21-23, additionally comprising the step of removing the lid from the container between step b) and step c).

27. Method according to any of claims 21-23, additionally comprising the step of removing the lid from the container between step c) and step d).
